**Europäisches Patentamt**

(19) **European Patent Office**

**Office européen des brevets**

(11) Publication number: **0 040 452**
**B1**

(12) **EUROPEAN PATENT SPECIFICATION**

(45) Date of publication of patent specification: **29.08.84**

(51) Int. Cl.³: **C 07 C 37/56,** C 07 C 39/04, B 01 J 23/89, B 01 J 23/84

(21) Application number: **81200516.3**

(22) Date of filing: **14.05.81**

(54) Method for the preparation of substituted or unsubstituted phenol and catalysts therefor.

(30) Priority: **16.05.80 NL 8002834**
**16.05.80 NL 8002833**

(43) Date of publication of application:
**25.11.81 Bulletin 81/47**

(45) Publication of the grant of the patent:
**29.08.84 Bulletin 84/35**

(84) Designated Contracting States:
**BE DE FR GB IT NL**

(56) References cited:
**FR-A-2 409 086**
**GB-A- 970 683**
**GB-A- 978 918**
**US-A-2 727 924**
**US-A-3 371 110**

**CHEMICAL ABSTRACTS, vol. 74, no. 19, 10th May 1971, page 468, no. 99551t, Columbus, Ohio, U.S.A., TAN, HUNG-SAN: "Oxidation of aromatic acids"**
**CHEMICAL ABSTRACTS, vol. 91, no. 17, 22nd October 1979, page 591, no. 140048a, Columbus, Ohio, U.S.A., A. AMREI et al.: "Studies on oxidation of benzoic acid to phenol"**

(73) Proprietor: **STAMICARBON B.V.**
**Postbus 10**
**NL-6160 MC Geleen (NL)**

(72) Inventor: **van Geem, Paul Christiaan**
**C. Hugostraat 13**
**NL-6176 BS Spaubeek (NL)**
Inventor: **Teunissen, Antonius Jacobus**
**v. Ostadestraat 42**
**NL-6165 XM Geleen (NL)**

(74) Representative: **Hoogstraten, Willem Corneiis Roeland et al**
**OCTROOIBUREAU DSM Postbus 9**
**NL-6160 MA Geleen (NL)**

(56) References cited:
**CHEMICAL ABSTRACTS, vol. 78, no. 7, 19th February 1973, page 447, no. 42983p, Columbus, Ohio, U.S.A., N.L. D'YACHENKO et al.: "Oxidative decarboxylation of m-toluic acid into cresols"**

# O 040 452

**Description**

The invention relates to a method for the preparation of a substituted or unsubstituted phenol by oxidation of the corresponding substituted or unsubstituted benzoic acid in the gas phase in the presence of a copper containing catalyst and catalysts therefor. The invention particularly relates to the gas phase oxidation of unsubstituted benzoic acid to unsubstituted phenol.

Such a method and catalyst are known from the Dutch patent specification 107,561. This patent specification describes such a method and catalyst, in which the catalyst consists of copper oxide on a magnesium oxide carrier. The disadvantage of such a catalyst is that the activity and/or selectivity of it will strongly decrease after a very short time, for instance already after a few hours, so that the application of such a method for the preparation of phenols was economically unattainable.

So far all kinds of attempts have been made to come to an improved catalyst for the above-mentioned method. Thus it was suggested to replace a large part, often even far more than half, of the copper oxide. Such catalysts, however, show faults all of them. Thus no catalyst has so far been known to combine a good activity and selectivity with a long economic life.

The purpose of the invention is to provide a catalyst which does combine these properties.

A method according to the invention is characterized in that the catalyst contains:
A. copper and/or one or more copper compounds,
B. vanadium and/or one or more vanadium compounds and/or silver and/or one or more silver compounds, and
C. lithium and/or one or more lithium compounds and/or sodium and/or one or more sodium compounds and/or magnesium and/or one or more magnesium compounds with an atom ratio between vanadium and copper of at most 1:2, between silver and copper of at most 1:2, between lithium and copper of at most 5:1, between sodium and copper of at most 5:1 and between magnesium and copper of at most 5:1.

The catalyst used according to the invention preferably consists of an intimate mixture of CuO and $V_2O_5$ and/or $Ag_2O$ and $Li_2O$ and/or $Na_2O$ and/or MgO and, optionally, one or more other compounds. In addition the catalyst preferably contains 0—95% by weight of carrier material, more specifically 20—80% by weight of carrier material. Preferably a silicon-containing carrier material is applied.

A suitable method of preparation for the catalyst according to the invention is as follows.

The carrrier material is suspended in distilled water. Subsequently, at virtually room temperature, for instance 280—320 K, a solution of copper nitrate, a solution of ammonium metavanadate and/or a solution of silvernitrate and, optionally, one or more solutions of one or more cocatalyst metal-containing compounds are added to this suspension.

During firm stirring the metals are precipitated as hydroxide or oxide by adding, in drops, an equivalent quantity of a solution and/or a suspension of lithium and/or sodium and/or magnesium hydroxide. Subsequently the suspension is filtered. The filter cake is broken, dried and, for a certain length of time, for instance 1—50 hours and preferably for 10—25 hours, calcined at a temperature of, for instance, 500—1000 K and preferable of 500—750 K. Finally, the catalyst thus obtained is reduced in size, if necessary, with mechanical means and optionally sieved. The desired catalyst particle size depends on the kind and the size of the particular reactor in which the catalyst particles are applied.

The method according to the invention is carried out as follows.

1 mol part of substituted or unsubstituted benzoic acid, with 1—100 mol parts of water, for instance in the form of steam, and preferably 5—25 mol parts of water, and 0.05—5 mol parts of oxygen and preferably 0.1—2 mol parts of oxygen, for instance in the form of air, is brought into contact in the gas phase with a catalyst according to the invention at a temperature of 450—700 K, preferably 500—650 K, and at a pressure of preferably 50—2000 kPa, at which higher and lower pressures are not excluded as long as the gas phase is maintained in the reaction medium. Preferably a catalyst load of 0.05—5 parts by weight of substituted or unsubstituted benzoic acid per part by weight of catalyst is applied per hour, specifically 0.1—2 parts by weight of substituted or unsubstituted benzoic acid per part by weight of catalyst per hour. Higher and lower catalyst loads can also be applied, but are, for economic reasons, less attractive.

The gas phase oxidation according to the invention can be effected in all kinds of reactors. Very well applicable are fixed-bed reactors and particularly fluid bed reactors. In a fixed-bed reactor preference is given to the use of catalyst particles with a diameter of between 1 and 10 mm. In a fluid bed reactor preference is given to the use of catalyst particles containing a carrier with a high specific surface, preferably larger than 100 m²/g, more specifically larger than 200 m²/g, and which particles preferably have a diameter of between 20 and 500 $\mu$m.

A very suitable method to carry out the gas phase oxidation according to the invention in a fluid bed reactor is as follows.

The fluidization of the catalyst particles is effected by the supply of oxidizing gas and steam. Thus

2

a fluid bed is formed with a height h. The substituted or unsubstituted benzoic acid to be oxidized is fed, in gaseous state, into the fluid bed at a place separate from the oxidizing gas supply, at a height preferably between 1/4 and 3/4 h.

The reaction mixture formed during the gas phase oxidation can be received and condensed. The liquid reaction mixture thus formed can then be separated, as known in the art, into the various components, for instance by distillation. Moreover, instead of ordinary condensation of the reaction gases, fractional condensation can be applied. Thus a separation by distillation can afterwards become superfluous.

The invention also includes catalysts for the catalysis of the gas phase oxidation of substituted or unsubstituted benzoic acid to the corresponding substituted or unsubstituted phenol. These catalysts contain 1000 parts of copper and/or one or more copper compounds, particularly copper oxide, (calculated as Cu atoms) together with 4—100 parts of vanadium and/or one or more vanadium compounds (calculated as V atoms) and/or 2—250 parts of silver and/or one or more silver compounds (calculated as Ag atoms) and 5—2000 parts of lithium and/or one or more lithium compounds (calculated as Li atoms) and/or 5—2000 parts of sodium and/or one or more sodium compounds (calculated as Na atoms) and/or 5—2000 parts of magnesium and/or one or more magnesium compounds (calculated as Mg atoms). These catalysts preferably contain 0—95% by weight of carrier material, more specifically 20—80% by weight of carrier material. Preferably a silicon-containing carrier material is applied. By preference does this carrier material have a specific surface larger than 100 m²/g, more particularly larger than 200 m²/g.

It is remarked that from GB—A—978,918 it is known to convert a benzene carboxylic acid into a phenolic compound using a catalyst containing copper, a transition metal from group III to VII of the Periodic System and an activating metal chosen from cobalt, the alkali metals and the alkaline earth metals. The atomic ratio between copper and transition metal ranges from 20:1 to 1:20 and between activating metal and copper is at least 2:1. However, the limits of present invention and the very specific catalyst composition to be applied can never be derived from this GB—A—978,918.

The invention is further elucidated by means of the following non-restrictive examples and comparative experiments.

Examples and comparative experiments

In the table below examples 1 up to and including 21 and comparative experiments A up to and including G are given. Examples 1 up to and including 20 and comparative experiments A up to and including F have been carried out in a fluid bed reactor as described above, into which reactor the benzoic acid was fed at about ½h at virtually atmospheric pressure. The example 21 and comparative experiment G have been carried out in a fixed-bed reactor as described above, again at virtually atmospheric pressure.

The load during the experiments was each time about 0.4 g benzoic acid per g catalyst per hour, the molar ratio $H_2O$: benzoic acid about 10:1 to 20:1 and $O_2$: benzoic acid about 0.2.

After 1 hour, 24 hours, 96 hours and 120 hours samples of the reactor gases released were cooled, homogenized with dimethylformamide and analyzed. The results of these analyses are given in the table.

The catalyst used in the gas phase oxidation process had been prepared as described above. In this preparation a calcination time of 16 hours was observed. Besides the metal compounds, the catalyst consisted of Aerosil, a silicon-containing carrier material with a specific surface larger than 200 m²/g.

The table below shows the following:

| Column I | number of example or comparative experiment |
|---|---|
| II | % by weight of Cu in catalyst |
| III | % by weight of V in catalyst |
| IV | % by weight of Ag in catalyst |
| V | % by weight of Li in catalyst |
| VI | % by weight of Na in catalyst |
| VII | % by weight of Mg in catalyst |
| VIII | duration of the experiment till the measurement in hours |
| IX | conversion in moles % |
| X | phenol selectivity in moles % |
| XI | benzene selectivity in moles % |
| XII | diphenylether selectivity in moles % |
| XIII | calcination temperature in K |
| XIV | reaction temperature in K |

The remainder of the reaction gas consisted of combustion products.

| I | II | III | IV | V | VI | VII | VIII | IX | X | XI | XII | XIII | XIV |
|---|---|---|---|---|---|---|---|---|---|---|---|---|---|
| 1 | 28,3 | 1,2 | | 1,5 | | | 1 | 35,1 | 79,8 | 6,3 | 1,7 | 585 | 570 |
| | | | | | | | 24 | 35,1 | 86,4 | 4,8 | 1,4 | | |
| | | | | | | | 96 | 34,9 | 82,3 | 6,2 | 1,8 | | |
| | | | | | | | 120 | 34,1 | 80,1 | 7,1 | 1,9 | | |
| 2 | 28,7 | 4,0 | | | 1,2 | | 1 | 20,5 | 75,3 | 5,3 | 5,2 | 700 | 540 |
| | | | | | | | 24 | 20,8 | 74,1 | 5,5 | 7,2 | | |
| | | | | | | | 96 | 21,0 | 73,4 | 5,7 | 8,8 | | |
| | | | | | | | 120 | 20,1 | 72,5 | 6,0 | 9,3 | | |
| 3 | 34,0 | 1,8 | | | 0,9 | | 1 | 20,9 | 72,1 | 4,4 | 5,9 | 700 | 540 |
| | | | | | | | 24 | 21,9 | 72,1 | 5,9 | 8,9 | | |
| | | | | | | | 96 | 23,5 | 71,8 | 6,3 | 11,0 | | |
| | | | | | | | 120 | 20,9 | 70,7 | 7,8 | 13,8 | | |
| 4 | 30,1 | 0,6 | | | 9,8 | | 1 | 44,7 | 77,9 | 8,1 | 8,2 | 575 | 540 |
| | | | | | | | 24 | 39,2 | 76,1 | 8,1 | 8,0 | | |
| | | | | | | | 96 | 31,0 | 74,8 | 8,2 | 8,5 | | |
| | | | | | | | 120 | 26,8 | 73,8 | 9,0 | 10,1 | | |
| 5 | 33,6 | 1,8 | | | 0,8 | | 1 | 25,2 | 77,1 | 5,8 | 6,7 | 575 | 540 |
| | | | | | | | 24 | 24,7 | 76,0 | 7,4 | 8,2 | | |
| | | | | | | | 96 | 24,1 | 73,5 | 6,5 | 10,2 | | |
| | | | | | | | 120 | 23,9 | 72,1 | 8,0 | 11,3 | | |
| 6 | 31,2 | 2,0 | | | | 5,8 | 1 | 15,3 | 73,1 | 8,5 | 3,1 | 580 | 570 |
| | | | | | | | 24 | 14,9 | 72,1 | 9,3 | 4,0 | | |
| | | | | | | | 96 | 14,6 | 71,8 | 10,1 | 5,9 | | |
| | | | | | | | 120 | 14,1 | 72,0 | 11,0 | 4,9 | | |
| 7 | 27,4 | | 0,4 | 1,7 | | | 1 | 50,5 | 81,1 | 8,5 | 3,0 | 580 | 573 |
| | | | | | | | 24 | 48,1 | 80,2 | 8,9 | 3,2 | | |
| | | | | | | | 96 | 45,0 | 78,9 | 9,1 | 5,0 | | |
| | | | | | | | 120 | 43,7 | 78,1 | 9,5 | 4,8 | | |
| 8 | 27,6 | | 9,3 | | 4,5 | | 1 | 33,1 | 73,8 | 7,6 | 5,6 | 700 | 555 |
| | | | | | | | 24 | 33,0 | 73,7 | 7,9 | 6,0 | | |
| | | | | | | | 96 | 32,9 | 73,4 | 8,9 | 6,2 | | |
| | | | | | | | 120 | 32,0 | 73,2 | 9,0 | 6,3 | | |
| 9 | 27,6 | | 9,3 | | 4,5 | | 1 | 40,6 | 80,1 | 6,9 | 5,0 | 575 | 555 |
| | | | | | | | 24 | 37,8 | 78,2 | 6,9 | 6,0 | | |
| | | | | | | | 96 | 35,7 | 77,1 | 6,2 | 6,7 | | |
| | | | | | | | 120 | 35,1 | 76,9 | 6,3 | 6,9 | | |
| 10 | 32,0 | | 0,1 | | 3,4 | | 1 | 42,5 | 81,9 | 5,4 | 6,9 | 700 | 575 |
| | | | | | | | 24 | 32,7 | 71,0 | 13,7 | 6,2 | | |
| | | | | | | | 96 | 24,8 | 71,1 | 15,1 | 6,3 | | |
| | | | | | | | 120 | 23,2 | 70,1 | 15,3 | 5,8 | | |
| 11 | 31,2 | | 0,3 | | | 6,0 | 1 | 18,1 | 80,0 | 8,5 | 5,1 | 580 | 570 |
| | | | | | | | 24 | 17,3 | 79,1 | 9,1 | 6,0 | | |
| | | | | | | | 96 | 16,3 | 78,0 | 10,0 | 6,1 | | |
| | | | | | | | 120 | 16,1 | 77,1 | 11,0 | 7,0 | | |
| 12 | 31,2 | 1,2 | 0,5 | 3,8 | | | 1 | 43,7 | 88,6 | 4,0 | 1,9 | 585 | 570 |
| | | | | | | | 24 | 46,1 | 86,8 | 3,8 | 1,5 | | |
| | | | | | | | 96 | 45,0 | 84,1 | 5,1 | 2,2 | | |
| | | | | | | | 120 | 44,2 | 83,2 | 6,2 | 3,0 | | |
| 13 | 30,0 | 1,3 | 0,4 | 1,9 | | | 1 | 48,5 | 84,7 | 6,4 | 1,5 | 590 | 570 |
| | | | | | | | 24 | 47,2 | 87,9 | 4,8 | 1,3 | | |
| | | | | | | | 96 | 47,8 | 83,5 | 6,1 | 2,0 | | |
| | | | | | | | 120 | 46,1 | 84,1 | 7,0 | 2,5 | | |
| 14 | 31,8 | 1,1 | 0,3 | | 1,2 | | 1 | 21,7 | 80,8 | 4,2 | 7,2 | 575 | 540 |
| | | | | | | | 24 | 19,3 | 79,8 | 4,5 | 8,3 | | |
| | | | | | | | 96 | 19,7 | 79,5 | 4,6 | 8,3 | | |
| | | | | | | | 120 | 21,4 | 75,8 | 6,2 | 10,1 | | |
| 15 | 31,8 | 1,1 | 0,3 | | 1,2 | | 1 | 47,5 | 78,8 | 4,3 | 7,0 | 575 | 590 |
| | | | | | | | 24 | 52,4 | 79,3 | 4,6 | 8,0 | | |
| | | | | | | | 96 | 45,4 | 80,4 | 5,1 | 4,9 | | |
| | | | | | | | 120 | 39,5 | 79,3 | 3,9 | 3,0 | | |

| I | II | III | IV | V | VI | VII | VIII | IX | X | XI | XII | XIII | XIV |
|---|---|---|---|---|---|---|---|---|---|---|---|---|---|
| 16 | 33,5 | 0,7 | 0,2 | | 0,9 | | 1 | 22,3 | 76,1 | 7,6 | 8,0 | 575 | 565 |
| | | | | | | | 24 | 21,9 | 75,2 | 10,8 | 7,7 | | |
| | | | | | | | 96 | 21,7 | 74,1 | 12,9 | 7,4 | | |
| | | | | | | | 120 | 21,0 | 72,4 | 14,8 | 6,7 | | |
| 17 | 31,1 | 0,4 | 1,1 | | 1,1 | | 1 | 15,2 | 75,4 | 3,7 | 8,0 | 575 | 540 |
| | | | | | | | 24 | 16,2 | 74,9 | 5,2 | 7,8 | | |
| | | | | | | | 96 | 15,1 | 74,7 | 6,1 | 7,6 | | |
| | | | | | | | 120 | 14,9 | 74,3 | 7,1 | 7,2 | | |
| 18 | 31,1 | 0,4 | 1,1 | | 1,1 | | 1 | 37,1 | 75,2 | 13,8 | 7,5 | 575 | 565 |
| | | | | | | | 24 | 35,7 | 73,6 | 16,1 | 6,8 | | |
| | | | | | | | 96 | 36,9 | 74,7 | 14,7 | 6,2 | | |
| | | | | | | | 120 | 35,0 | 74,6 | 15,7 | 6,6 | | |
| 19 | 32,0 | 1,1 | 0,4 | | | 2,0 | 1 | 30,1 | 77,1 | 10,5 | 8,0 | 575 | 590 |
| | | | | | | | 24 | 30,2 | 78,0 | 11,0 | 7,5 | | |
| | | | | | | | 96 | 29,8 | 77,5 | 10,8 | 8,9 | | |
| | | | | | | | 120 | 27,5 | 77,0 | 10,9 | 9,5 | | |
| 20 | 30,6 | 1,3 | 0,4 | 1,2 | 4,3 | | 1 | 46,7 | 84,4 | 6,6 | 3,3 | 585 | 575 |
| | | | | | | | 24 | 41,6 | 86,3 | 4,6 | 3,3 | | |
| | | | | | | | 96 | 38,1 | 86,4 | 4,5 | 3,4 | | |
| | | | | | | | 120 | 37,7 | 85,9 | 5,1 | 3,3 | | |
| 21 | 30,6 | 1,3 | 0,4 | 1,2 | 4,3 | | 1 | 32,1 | 81,3 | 4,1 | 5,1 | 585 | 575 |
| | | | | | | | 24 | 31,6 | 80,1 | 3,6 | 6,0 | | |
| | | | | | | | 96 | 30,5 | 81,2 | 3,9 | 5,9 | | |
| | | | | | | | 120 | 29,8 | 79,5 | 4,2 | 4,8 | | |
| A | 30,1 | | | | | | 1 | 10,0 | 48,4 | 7,4 | 1,6 | 585 | 540 |
| | | | | | | | 24 | 5,2 | 36,8 | 7,9 | 5,7 | | |
| | | | | | | | 96 | 3,6 | 30,1 | 8,0 | 10,9 | | |
| | | | | | | | 120 | 3,0 | 25,9 | 8,1 | 12,8 | | |
| B | 28,8 | | | 1,8 | | | 1 | 40,3 | 77,6 | 11,3 | 3,1 | 585 | 575 |
| | | | | | | | 24 | 31,5 | 75,3 | 13,0 | 2,5 | | |
| | | | | | | | 96 | 25,7 | 70,1 | 18,1 | 3,7 | | |
| | | | | | | | 120 | 20,8 | 61,9 | 22,0 | 5,6 | | |
| C | 33,7 | | | | 3,9 | | 1 | 20,2 | 70,8 | 22,3 | 2,8 | 575 | 540 |
| | | | | | | | 24 | 20,1 | 60,3 | 28,5 | 3,9 | | |
| | | | | | | | 96 | 19,8 | 51,2 | 35,1 | 5,8 | | |
| | | | | | | | 120 | 19,7 | 35,9 | 40,7 | 8,9 | | |
| D | 31,2 | | | | | 6,0 | 1 | 25,0 | 72,8 | 15,1 | 3,9 | 580 | 565 |
| | | | | | | | 24 | 21,0 | 67,9 | 17,9 | 4,1 | | |
| | | | | | | | 96 | 18,1 | 60,1 | 25,1 | 5,9 | | |
| | | | | | | | 120 | 17,0 | 55,1 | 30,7 | 8,7 | | |
| E | 9,9 | 15,8 | | | 4,0 | | 1 | 6,9 | 43,0 | 6,5 | 0,6 | 700 | 540 |
| | | | | | | | 24 | 6,1 | 35,1 | 6,4 | 1,3 | | |
| | | | | | | | 96 | 5,4 | 29,7 | 6,6 | 1,9 | | |
| | | | | | | | 120 | 4,8 | 25,1 | 6,7 | 2,6 | | |
| F | 10,9 | | 18,8 | | 4,0 | | 1 | 31,9 | 46,5 | 27,4 | 8,4 | 590 | 570 |
| | | | | | | | 24 | 28,1 | 34,9 | 33,2 | 6,6 | | |
| | | | | | | | 96 | 28,4 | 33,7 | 35,6 | 5,7 | | |
| | | | | | | | 120 | 27,5 | 31,6 | 36,4 | 7,8 | | |
| G | 33,7 | | | | 3,9 | | 1 | 16,2 | 72,3 | 23,1 | 3,7 | 575 | 540 |
| | | | | | | | 24 | 15,9 | 61,2 | 29,0 | 4,8 | | |
| | | | | | | | 96 | 14,3 | 44,9 | 35,6 | 5,8 | | |
| | | | | | | | 120 | 13,9 | 31,0 | 45,9 | 5,8 | | |

## Claims

1. Method for the preparation of substituted or unsubstituted phenol by oxidation of the corresponding substituted or unsubstituted benzoic acid in the gas phase in the presence of a copper containing catalyst, characterized in that, the catalyst contains:

A. copper and/or one or more copper compounds,

B. vanadium and/or one or more vanadium compounds and/or silver and/or one or more silver compounds, and

5

C. lithium and/or one or more lithium compounds and/or sodium and/or one or more sodium compounds and/or magnesium and/or one or more magnesium compounds

with an atom ratio between vanadium and copper of at most 1:2 between silver and copper of at most 1:2, between lithium and copper of at most 5:1, between sodium and copper of at most 5:1 and between magnesium and copper of at most 5:1.

2. Method according to claim 1, characterized in that a vanadium-copper atom ratio of between 1:250 and 1:10 is applied.

3. Method according to claim 1 or 2, characterized in that a silver-copper atom ratio of between 1:500 and 1:4 is applied.

4. Method according to any one of the claims 1—3, characterized in that a lithium-copper atom ratio of between 1:200 and 2:1 is applied.

5. Method according to any one of the claims 1—4, characterized in that a sodium-copper an atom ratio of between 1:200 and 2:1 is applied.

6. Method according to any one of the claims 1—5, characterized in that a magnesium-copper atom ratio of between 1:200 and 2:1 is applied.

7. Method according to any one of the claims 1—6, characterized in that the said gas phase oxidation is carried out in a fluid bed reactor.

8. Method according to claim 7, characterized in that the substituted or unsubstituted benzoic acid to be oxidized is fed to the fluid bed separately from the supply for oxygen-containing gas at a height of between 1/4 and 3/4 parts of the total height of the bed.

9. Catalyst for the catalysts of the gas phase oxidation of substituted and unsubstituted benzoic acid to the corresponding substituted or unsubstituted phenol, characterized in that this catalyst contains 1000 parts of copper and/or one or more copper compounds (calculated as Cu atoms) together with 4—100 parts of vanadium and/or one of more vanadium compounds (calculated as V atoms) and/or 2—250 parts of silver and/or one or more silver compounds (calculated as Ag atoms) and 5—2000 parts of lithium and/or one or more lithium compounds (calculated as Li atoms) and/or 5—2000 parts of sodium and/or one or more sodium compounds (calculated as Na atoms) and/or 5—2000 parts of magnesium and/or one or more magnesium compounds (calculated as Mg atoms).

10. Method for the gas phase oxidation of substituted or unsubstituted benzoic acid in substance as described above and elucidated by means of the examples.

11. Catalyst for the gas phase oxidation of substituted or unsubstituted benzoic acid in substance as described above and prepared in a manner as described above and elucidated by means of the examples.

12. Substituted or unsubstituted phenol prepared by gas phase oxidation of the corresponding substituted or unsubstituted benzoic acid according to a method according to any one of the claims 1—8 and 10 catalyzed with a catalyst according to any one of the claims 9 and 11.

**Patentansprüche**

1. Verfahren zur Herstellung von substituiertem oder unsubstituiertem Phenol durch Oxydation der entsprechenden substituierten oder unsubstituierten Benzoesäure in der Gasphase in Anwesenheit, eines Kupfer enthaltenden Katalysators, dadurch gekennzeichnet, daß der Katalysator enthält:

A. Kupfer und/oder eine bzw. mehrere Kupferverbindungen,

B. Vanadium und/oder eine bzw. mehrere Vanadiumverbindungen und/oder Silber und/oder eine bzw. mehrere Silberverbindungen sowie

C. Lithium und/oder eine oder mehrere Lithiumverbindungen und/oder Natrium und/oder eine bzw. mehrere Natriumverbindungen und/oder Magnesium und/oder eine bzw. mehrere Magnesiumverbindungen

mit einem Atomverhältnis zwischen Vanadium und Kupfer von maximal 1:2, zwischen Silber und Kupfer von maximal 1:2, zwischen Lithium und Kupfer von maximal 5:1, zwischen Natrium und Kupfer von maximal 5:1 und zwischen Magnesium und Kupfer von maximal 5:1.

2. Verfahren nach Anspruch 1, dadurch gekennzeichnet, daß ein Atomverhältnis zwischen Vanadium und Kupfer von 1:250 bis 1:10 verwendet wird.

3. Verfahren nach Anspruch 1 oder 2, dadurch gekennzeichnet, daß ein Atomverhältnis zwischen Silber und Kupfer von 1:500 bis 1:4 verwendet wird.

4. Verfahren nach einem der Ansprüche 1—3, dadurch gekennzeichnet, daß ein Atomverhältnis zwischen Lithium und Kupfer von 1:200 bis 2:1 verwendet wird.

5. Verfahren nach einem der Ansprüche 1—4, dadurch gekennzeichnet, daß ein Atomverhältnis zwischen Natrium und Kupfer von 1:200 bis 2:1 verwendet wird.

6. Verfahren nach einem der Ansprüche 1—5, dadurch gekennzeichnet, daß ein Atomverhältnis zwischen Magnesium und Kupfer von 1:200 bis 2:1 verwendet wird.

## 0 040 452

7. Verfahren nach einem der Ansprüche 1—6, dadurch gekennzeichnet, daß die genannte Oxydation in der Gasphase in einem Fließbettreaktor durchgeführt wird.

8. Verfahren nach Anspruch 7, dadurch gekennzeichnet, daß die zu oxydierende substituierte oder unsubstituierte Benzoesäure dem Fließbett getrennt von der Zufuhr für sauerstoffhaltiges Gas in einer Höhe von 1/4 bis 3/4 der Gesamthöhe des Fließbettes zugeführt wird.

9. Katalysator für die Katalyse der Oxydation von substituierter oder unsubstituierter Benzoesäure in der Gasphase zum entsprechenden substituierten oder unsubstituierten Phenol, dadurch gekennzeichnet, daß dieser Katalysator 1000 Teile Kupfer und/oder eine bzw. mehrere Kupferverbindungen (berechnet als Cu-Atome) enthält, und zwar zusammen mit 4—100 Teilen Vanadium und/oder einer bzw. mehreren Vanadiumverbindungen (berechnet als V-Atome) und/oder 2—250 Teilen Silber und/oder einer bzw. mehreren Silberverbindungen (berechnet als Ag-Atome) und 5—2000 Teilen Lithium und/oder einer bzw. mehreren Lithiumverbindungen (berechnet als Li-Atome) und/oder 5— 2000 Teilen Natrium und/oder einer bzw. mehreren Natriumverbindungen (berechnet als Na-Atome) und/oder 5—2000 Teilen Magnesium und/oder einer oder mehreren Magnesiumverbindungen (berechnet als Mg-Atome).

10. Verfahren zur Oxydation von substituierter oder unsubstituierter Benzoesäure in der Gasphase, im wesentlichen wie oben beschrieben und durch die Beispiele erläutert.

11. Katalysator für die Oxydation von substituierter oder unsubstituierter Benzoesäure in der Gasphase, im wesentlichen wie oben beschrieben und hergestellt auf eine Weise, wie oben beschrieben und durch die Beispiele erläutert.

12. Substituiertes oder unsubstituiertes Phenol, hergestellt durch Oxydation der entsprechenden substituierten oder unsubstituierten Benzoesäure in der Gasphase gemäß einem der Ansprüche 1—8 und 10, katalysiert mit einem Katalysator gemäß einem der Ansprüche 9 und 11.

**Revendications**

1. Procédé de préparation de phénol substitué ou non substitué par oxydation en phase gazeuse d'acide benzoïque substitué ou non substitué correspondant, en présence d'un catalyseur contenant du cuivre, caractérisé en ce que le catalyseur contient:

A. du cuivre et/ou un ou plusieurs composés de cuivre
B. du vanadium et/ou un ou plusieurs composés de vanadium et/ou de l'argent et/ou un ou plusieurs composés d'argent, et
C. du lithium et/ou un ou plusieurs composés de lithium et/ou du sodium et/ou un ou plusieurs composés de sodium et/ou du magnésium et/ou un ou plusieurs composés de magnésium

avec un rapport atomique entre le vanadium et le cuivre de tout au plus 1:2, alors que celui entre l'argent et le cuivre est de tout au plus 1:2, celui entre le lithium et le cuivre de tout au plus 5:1, celui entre le sodium et le cuivre de tout au plus 5:1 et celui entre le magnésium et le cuivre de tout au plus 5:1.

2. Procédé selon la revendication 1, caractérisé en ce qu'on applique un rapport atomique entre le vanadium et le cuivre situé entre 1:250 et 1:10.

3. Procédé selon l'une des revendications 1 à 2, caractérisé en ce qu'on applique un rapport atomique entre l'argent et le cuivre situé entre 1:500 et 1:4.

4. Procédé selon l'une des revendications 1 à 3, caractérisé en ce qu'on applique un rapport atomique lithium-cuivre situé entre 1:200 et 2:1.

5. Procédé selon l'une des revendications 1 à 4, caractérisé en ce qu'on applique un rapport atomique sodium-cuivre situé entre 1:200 et 2:1.

6. Procédé selon l'une des revendications 1 à 5, caractérisé en ce qu'on applique un rapport atomique magnésium-cuivre situé entre 1:200 et 2:1.

7. Procédé selon l'une des revendications 1 à 6, caractérisé en ce que ladite oxydation en phase gazeuse est exécutée dans un réacteur à lit fluidisé.

8. Procédé selon la revendication 7, caractérisé en ce que l'acide benzoïque substitué ou non substitué qu'il faut oxyder est introduit dans le lit fluidisé à un endroit séparé de celui de l'introduction du gaz contenant de l'oxygène, à une hauteur située entre 1/4 et 3/4 de la hauteur totale de lit.

9. Catalyseur pour la catalyse de l'oxydation en phase gazeuse d'acide benzoïque substitué ou non substitué en le phénol substitué ou non substitué correspondant, caractérisé en ce que ce catalyseur contient 1000 parties de cuivre et/ou d'un ou plusieurs composés de cuivre (calculés comme atomes de Cu) ensemble avec 4—100 parties de vanadium et/ou d'un ou plusieurs composés de vanadium (calculés comme atomes de V) et/ou 2—250 parties d'argent et/ou d'un ou plusieurs composés d'argent (calculés comme atomes d'Ag) et 5—2000 parties de lithium et/ou d'un ou plusieurs composés de lithium (calculés comme atomes de Li) et/ou 5—2000 parties de sodium et/ou d'un ou plusieurs composés de sodium (calculés comme atomes de Na) et/ou 5—2000 parties de magnésium et/ou d'un ou plusieurs composés de magnésium (calculés comme atomes de Mg).

**0 040 452**

10. Procédé d'oxydation en phase gaseuse d'acide benzoïque substitué ou non substitué, essentiellement comme décrit ci-dessus et expliqué à l'aide des exemples.

11. Catalyseur pour l'oxydation en phase gazeuse d'acide benzoïque substitué ou non substitué, essentiellement comme décrit ci-dessus et préparé de la manière décrite ci-dessus et expliqué à l'aide des exemples.

12. Phénol substitué ou non substitué préparé par l'oxydation en phase gazeuse de l'acide benzoïque substitué ou non substitué correspondant, selon le procédé de l'une des revendications 1 à 8 et 10, et catalysé avec un catalyseur selon l'une des revendications 9 et 11.